**Europäisches Patentamt**

**European Patent Office**

**Office européen des brevets**

(19)

(11) Veröffentlichungsnummer: **0 099 483**
**B1**

(12) # EUROPÄISCHE PATENTSCHRIFT

(45) Veröffentlichungstag der Patentschrift:
08.04.87

(51) Int. Cl.⁴: **A 61 B 10/00**

(21) Anmeldenummer: **83106118.9**

(22) Anmeldetag: **22.06.83**

(54) Ultraschall-Tomographiegerät.

(30) Priorität: **30.06.82 DE 3224412**

(43) Veröffentlichungstag der Anmeldung:
**01.02.84 Patentblatt 84/5**

(45) Bekanntmachung des Hinweises auf die Patenterteilung:
**08.04.87 Patentblatt 87/15**

(84) Benannte Vertragsstaaten:
**DE FR GB NL**

(56) Entgegenhaltungen:
**EP-A-0 032 751**
**WO-A-80/00193**
**FR-A-2 492 653**
**US-A-4 074 564**
**US-A-4 103 677**

(73) Patentinhaber: **Siemens Aktiengesellschaft Berlin und München, Wittelsbacherplatz 2, D-8000 München 2 (DE)**

(72) Erfinder: **Hassler, Dieter, Flurweg 3, D-8521 Uttenreuth (DE)**
Erfinder: **Trautenberg, Elmar, Dr., Steinacher Strasse 32, D-8510 Fürth- Stadeln (DE)**

## Beschreibung

Die Erfindung bezieht sich auf ein Ultraschall-Tomographiegerät gemäß den Merkmalen des Oberbegriffs des Patentanspruchs 1.

Ultraschall-Tomographiegeräte lassen sich sowohl nach der Transmissions- als auch nach der Reflexionsmethode betreiben. Ein Ultraschall-Tomographiegerät für Transmissions-Tomographie (UCTT) ist z.B. aus der US-PS 4,105,018 vorbekannt. Ultraschall-Tomographiegeräte für Reflexions-Tomographie (UCTR) sind beispielsweise bekannt durch den Aufsatz "Resolution And Image Quality By Ultrasonic Echo Tomography: Experimental Approach" von E.Hundt, G.Maderlechner, E.Kronmüller und E.Trautenberg im Tagungsbericht "Fifth International Symposium on Ultrasonic Imaging and Tissue Characterization And Second International Symposium on Ultrasonic Materials Characterization", June 1-6, 1980, Seite 7 oder auch durch den Aufsatz "Ultrasonic Reflectivity Tomography: Reconstruction With Circular Transducer Arrays" von Stephen J. Norton und Melvin Linzer aus "Ultrasonic Imaging 1", 1979, Seiten 154-184.

Die bekannten Ultraschall-Tomographiegeräte erlauben bei der Untersuchung, z.B. der weiblichen Brust, keine brustwandnahe Abtastung zwecks Erzeugung eines brustwandnahen Koronar-Schnittbildes, da wegen der räumlichen Abmessungen das Ultraschall-Sende-/Empfangssystem nicht nahe genug an die Brustwand herangeführt werden kann. Dasselbe gilt im Prinzip auch für das Ultraschall-Abtastgerät nach der US-PS 3,990,300, das mit einer Mehrzahl von einzelnen Sektor-Abtastern Sagittal-Schnittbilder erzeugt. Bei diesem Gerät sind die einzelnen Sektor-Abtaster auf einem Bogensegment angeordnet, das mit Hilfe eines Schlittens in horizontaler und vertikaler Richtung relativ zum Untersuchungsobjekt bewegbar ist. Eine Rotation um das Untersuchungsobjekt ist nicht vorgesehen.

Ein Ultraschallgerät mit den Merkmalen des Oberbegriffs des Anspruchs 1 ist aus der EP-A 0,032,751, insbesondere Fig. 2 und 3, bekannt. Bei diesem Gerät dreht sich ein halbkreisförmiger Haltearm, der einen Ultraschallkopf trägt, um eine Drehachse, die senkrecht durch eine Untersuchungsöffnung und damit durch die weibliche Brust verläuft. Der Bügel oder Haltearm ist weiterhin um eine Achse drehbar, die parallel zur Ebene des Patientenlagerungstisches, in dem sich die Untersuchungsöffnung befindet, verläuft. Bei diesem Gerät lassen sich somit Serien von Sonogrammschnittbildern der Brust aufnehmen, die in ihren Ebenen gegeneinander um vorgegebene Winkelbeträge um die Achse senkrecht durch den Mittelpunkt der Untersuchungsöffnung gedreht sind. Es handelt sich dabei also um Sagittal-Schnittebenen-Bilder. Koronar-Schnittbilder lassen sich auch mit diesem Gerät bei Bedarf nicht erzeugen.

Aufgabe der vorliegenden Erfindung ist es, ein Ultraschall-Tomographiegerät anzugeben, das ohne allzu grossen technischen Aufwand auf der Basis der Sagittal-Abtastung neben Sagittal-Schnittbildern bei Bedarf auch Koronar-Schnittbilder, insbesondere brustwandnahe Koronar-Schnittbilder, liefert.

Die Aufgabe wird erfindungsgemäß durch die kennzeichnenden Merkmale des Patentanspruchs 1 gelöst.

Die Erfindung ermöglicht den abschnittweisen Aufbau eines brustwandnahen Koronar-Schnittbildes aus einer Vielzahl von Sagittal-Schnitten mit Hilfe der Zeittorschaltung. Weitere, dazu parallele Koronar-Schnittbilder sind erhältlich, wenn an der Zeittorschaltung die Torzeiten entsprechend unterschiedlich eingestellt werden und/oder das Abtastsystem in seinem Abstand zur Brustwand geändert wird. Ebensogut können bei Bedarf auch reine Sagittal-Schnittbilder erzeugt werden. Bei geringem technischen Aufwand ergibt sich also eine Vielfalt von Anwendungsmöglichkeiten.

Weitere Vorteile und Einzelheiten der Erfindung ergeben sich aus der nachfolgenden Beschreibung mehrerer Ausführungsbeispiele anhand der Zeichnung und in Verbindung mit den Unteransprüchen.

Es zeigen:

Figur 1 ein erstes Ausführungsbeispiel in schematischem Aufbau,

Figur 2 eine schematische Skizze zur Verdeutlichung der Sagital-Schnittführung,

Figur 3 ein Prinzipschaltbild zur Anwendung bei der Erfindung,

Figur 4 ein zweites Ausführungsbeispiel der Erfindung und

Figur 5 ein drittes Ausführungsbeispiel der Erfindung.

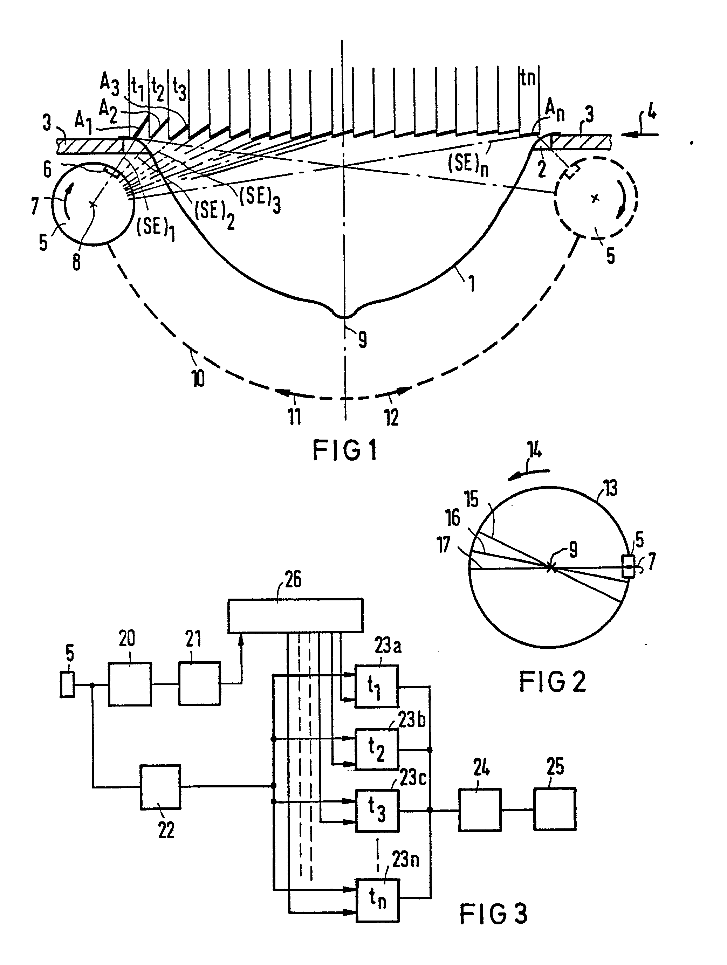

In Figur 1 ragt eine weibliche Brust 1 als Untersuchungsobjekt durch ein Applikationsloch 2 einer Platte 3, die z.B. Bestandteil eines Patiententisches ist, auf dem die Patientin liegt. Der Pfeil 4 deutet eine brustwandnahe koronare Schnittebene an, aus der ein Ultraschall-Sende-/Empfangssystem 5 z.B. ein brustwandnahes Koronar-Schnittbild liefern soll. Das Ultraschall-Sende-/Empfangssystem 5 besteht im vorliegenden Fall aus einem einzigen Sagittal-Abtaster. Der Sagittal-Abtaster ist ein mechanischer Sektor-Scanner mit Ultraschallwandler 6, der in Richtung des Rotationspfeiles 7 um die Rotationsochse 8 rotiert. Während der Rotation ergeben sich die strichpunktiert dargestellten, sich sektorförmig auffächernden Sende-/Empfangsrichtungen. Die für den Gewinn einer brustwandnahen Koronar-Schnittebene wesentlichen Sende-/Empfangsrichtungen sind mit $SE_1$ bis $SE_n$ dargestellt. Gleichzeitig mit der Sektor-Abtastung wird der Sagittal-Abtaster auch noch langsam um die durch die Brust 1 gehende Symmetrieachse als Drehachse 9 gedreht. Außerdem wandert der Sagittal-Abtaster entlang dem punktiert dargestellten Kreisbogen 10 in Richtung der Pfeile 11 und 12. Aufgrund dieser unterschiedlichen Bewegungsformen ergibt sich eine Sektor-Abtastung der Brust 1 aus einer beliebigen Zahl unterschiedlicher Winkelstellungen. In der Figur 1 sind lediglich zwei dieser Winkelstellungen (180°-Gegenlage) mit dem durchgezogen gezeichneten System 5 auf der linken Seite und dem punktiert gezeichneten System 5 auf der rechten Seite angedeutet.

Figur 2 zeigt eine Draufsicht auf das System der Figur 1. Demnach befindet sich der Sagital-Abtaster auf einem Kreisring 13, der in Richtung des Pfeiles 14 um die Drehachse 9 rotiert. Die Rotation des Abtasters in Richtung des Drehpfeiles 7 erzeugt die Sagital-Schnitte, von denen in der Figur 2 insgesamt drei mit 15, 16 und 17 angedeutet sind. Die Scheibenrotationsfrequenz ist sehr viel niedriger als die Frequenz, mit der der Sagittal-Abtaster geschwenkt oder gedreht wird. So beträgt z.B. die Drehgeschwindigkeit der Ringscheibe 13 zusammen mit dem Sagittal-Abtaster 0,1 Hz. Die Schwenk- oder Drehfrequenz des Sagittal-Abtasters liegt hingegen bei 3 bis 4 Hz.

Mit dem Ausführungsbeispiel der Figur 1 lassen sich Sagittal-Schnitte aus einer Vielzahl von Richtungen erzeugen, die nach entsprechender Umrechnung im Computer als sagittale CT-Bilder dargestellt werden können. Gemäß der Erfindung ist mit dem beschriebenen System aber auch der Aufbau von koronaren Schnittbildern, insbesondere von brustwandnahen Koronar-Schnittbildern, möglich. Dies geschieht mit Hilfe einer Zeittorschaltung, wie sie in der Figur 3 näher dargestellt und später beschrieben ist. Mit Hilfe dieser Zeittorschaltung lassen sich in aufeinanderfolgenden Zeitabschnitten t1, t2, t3 ... tn abschnittweise Signaldaten aus Bereichsabschnitten $A_1$, $A_2$,.... bis $A_n$ gemäß Figur 1 erfassen. Die Abschnitte $A_1$ bis $A_n$ nähern sehr gut die mit dem Pfeil 4 angedeutete brustwandnahe Koronar-Schnittebene an. Bei entsprechender Umrechnung der Daten in einem Rechner ergibt sich dann das Tomographiebild einer brustwandnahen Koronar-Ebene. Dazu parallele koronare Schnittebenen der Brust 1 erhält man, wenn an der Zeittorschaltung entsprechend unterschiedliche Zeiten eingestellt werden. Hier ergeben sich dann Abtastabschnitte, die sich zu beliebigen parallelen aren Schnittebenen zusammensetzen lassen. Das Bild der brustwandnahen Koronar-Schnittebene kann selbstverständlich auch aus anderen Positionen des Abtastsystems 5 auf dem Kreis 10 als in der dargestellten brustwandnahen Position ermittelt werden.

Figur 3 zeigt ein Prinzipschaltbild zur Steuerung des Ultraschall-Sende-/Empfangssystems 5 der Figur 1. Im Prinzipschaltbild ist der Sagittal-Abtaster wieder mit 5 bezeichnet. Der Sagittal-Abtaster 5 wird wie üblich von einem Hochfrequenzsender 20 mit vorgeschaltetem Taktgenerator 21 betrieben. Die während der Rotation des Systems aus der Brust 1 empfangenen Ultraschall-Echosignale werden einem Empfangsverstärker 22 zugeleitet. Von diesem gelangen sie dann auf eine Zeittorschaltung mit Zeittoren 23a bis 23n. Die Zeittore 23a bis 23n lassen nur während der in Figur 1 dargestellt Zeiten $t_1$ bis $t_n$ abschnittweise Echosignale zum nachfolgenden Computer 24 durch. Aus den so selektierten Daten errechnet der Computer 24 das angenäherte koronare brustwandnahe Schnittbild, das dann auf einer Anzeigevorrichtung 25 angezeigt wird. Mit 26 ist eine zentrale Steuereinrichtung für das System bezeichnet. Sollen neben Bildern der brustwandnahen Koronar-Schnittebene noch Bilder weiterer koronarer Schnittebenen gewonnen werden, so brauchen lediglich die Zeiten $t_1$ bis $t_n$ in der Zeittorschaltung so geändert zu werden, daß sich in Abwandlung von Figur 1 Echoabschnitte ergeben, die in angenäherten Ebenen liegen die parallel zur brustwandnahen koronaren Ebene liegen.

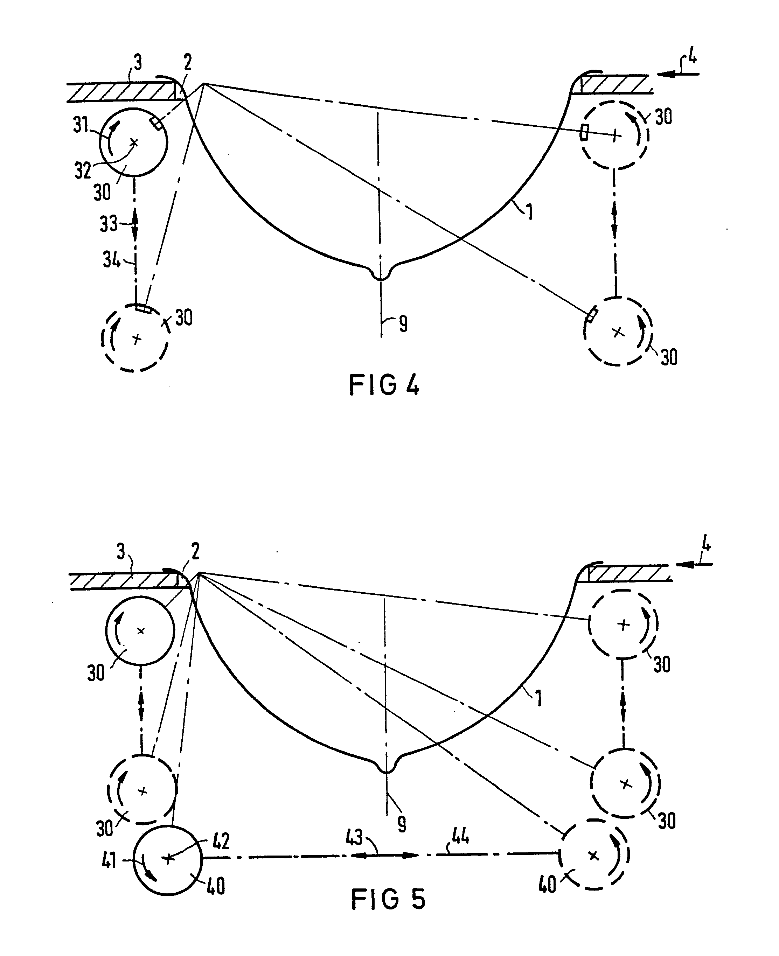

Figur 4 zeigt ein modifiziertes Ausführungsbeispiel dahingehend, daß ein Sagittal-Abtaster 30 wieder in Richtung des Rotationspfeiles 31 um eine Rotationsachse 32 rotiert. Im Unterschied zum Ausführungsbeispiel der Figur 1 vollführt dieser Sagittal-Abtaster 30 jedoch keine Bewegung auf einem zusätzlichen Kreisbogen 10 um die zu untersuchende Brust 1. Vielmehr wandert dieser Sagittal-Abtaster 30 in Richtung des Doppelpfeiles 33 entlang einer strichpunktierten Linie 34, die parallel zur Drehachse 9 verläuft.

Figur 5 zeigt eine Modifikation des Ausführungsbeispiels der Figur 4 dahingehend, daß zusätzlich zum Sagittal-Abtaster 30 ein zweiter Sagittal-Abtaster 40 vorhanden ist, der in Richtung des Doppelpfeiles 43 entlang der horizontalen strichpunktierten Linie 44 verschiebbar ist.

Es ist selbstverständlich, daß in sämtlichen Ausführungsbeispielen anstelle nur eines einzigen, sich in einer bestimmten Richtung bewegenden Sagittal-Abtasters auch deren mehrere vorhanden sein können, die entsprechend um das Untersuchungsobjekt herum verteilt angeordnet sind. Eine solche Ausführungsmöglichkeit fällt ebenfalls mit unter die Erfindung. Die Erfindung erlaubt ebenfalls den Einsatz beim Scatter-Scan, wo derselbe Objektpunkt aus unterschiedlichen Richtungen angeschallt und/oder abgefragt wird. Z.B. kann dazu in Figur 5 mit dem System 30 gesendet und mit dem System 40 empfangen werden. Sind am Umfang des Drehkreises 13 um die Brust 1 mehrere Systeme verteilt angeordnet, so kann jeweils ein beliebiges dieser Systeme Sender und ein anderes Empfänger sein.

## Patentansprüche

1. Ultraschall-Tomographiegerät mit einem Ultraschall-Sende-/Empfangssystem, das zur zeilenweisen Abtastung eines Untersuchungsobjektes (1) aus unterschiedlichen Winkelrichtungen ausgebildet und um eine Drehachse (9), die auf das Untersuchungsobjekt (1) gerichtet ist, drehbar ist, dadurch gekennzeichnet, daß das Ultraschall-Sende-/Empfangssystem ein Sagittal-Abtastsystem (5; 30; 40) mit sich in jeder Sagittal-Abtastebene (15, 16, 17) sektorförmig auffächernden Sende-/Empfangsrichtungen ist, dem eine Zeittorschaltung (23a bis 23n) zum abschnittweisen Erfassen von Signaldaten während vorgebbarer Zeiten ($t_1$ bis $t_n$) zugeordnet ist.

2. Ultraschall-Tomographiegerät nach Anspruch 1, dadurch gekennzeichnet, daß das Sagittal-Abtastsystem (5; 30; 40) wenigstens einen Sagittal-Abtaster umfaßt, der in vorgebbarem Abstand auf einem Weg (10; 34; 44) entlang der Kontur des Untersuchungsobjektes (1) verschiebbar ist.

3. Ultraschall-Tomographiegerät nach Anspruch 2, dadurch gekennzeichnet, daß das Sagittal-Abtastsystem einen Sagittal-Abtaster (5) umfaßt, der während seiner Drehung um die auf das Untersuchungsobjekt (1) gerichtete Drehachse (9) gleichzeitig einen Bogen (10), z. B. Kreisbogen, entlang der Kontur des Untersuchungsobjektes (1) beschreibt (Figur 1).

4. Ultraschall-Tomographiegerät nach Anspruch 2, dadurch gekennzeichnet, daß das Sagittal-Abtastsystem einen Sagittal-Abtaster (30) umfaßt, der während seiner Drehung um die auf das Untersuchungsobjekt (1) gerichtete Drehachse (9) auf einer Geraden (34) parallel zur Drehachse (9) längsverschiebbar ist (Figur 4).

5. Ultraschall-Tomographiegerät nach Anspruch 2, dadurch gekennzeichnet, daß das Sagittal-Abtastsystem einen Sagittal-Abtaster (40) umfaßt, der während seiner Drehung um die auf das Untersuchungsobjekt (1) gerichtete Drehachse (9) entlang einer Linie (44), die zur Drehachse (9) senkrecht und in vorgebbarem Höhenabstand zum Untersuchungsobjekt (1) liegt, verschiebbar angeordnet ist (Figur 5).

6. Ultraschall-Tomographiegerät nach einem der Ansprüche 1 bis 5, dadurch gekennzeichnet, daß das Ultraschall-Sende-/Empfangssystem mehrere Sagittal-Abtaster (30, 40) umfaßt, die sich abschnittweise in horizontalen und vertikalen Linien (34; 44) bewegen (Figur 5).

7. Ultraschall-Tomographiegerät nach einem der Ansprüche 1 bis 6, dadurch gekennzeichnet, daß das Sagittal-Abtastsystem (5; 30; 40) mechanische Sektor-Abtaster umfaßt.

## Revendications

1. Appareil de tomographie par ultrasons, avec un système d'émission/de réception d'ultrasons, qui est réalisé pour le balayage ligne par ligne d'un objet à examiner (1) à partir de différentes directions angulaires et qui est susceptible de tourner autour d'un axe de rotation (9) dirigé vers l'objet à examiner (1), caractérisé par le fait que le système d'émission/de réception est un système de balayage sagittal (5; 30; 40) comportant des dispositifs d'émission/de réception s'étalant en éventail et en forme de secteurs et auxquels est associé un circuit à portes de temps (23a à 23n) pour saisir, section par section, les données de signaux pendant des durées prédéterminées ($t_1$ à $t_n$).

2. Appareil de tomographie par ultrasons selon la revendication 1, caractérisé par le fait que le système de balayage sagittal (5; 30; 40) comporte au moins un détecteur sagittal qui, à une distance donnée à l'avance, est susceptible d'être déplacé sur une trajectoire (10; 34; 44) le long du contour de l'objet à examiner (1).

3. Appareil de tomographie par ultrasons selon la revendication 2, caractérisé par le fait que le système de balayage sagittal comporte un détecteur sagittal (5) qui, pendant sa rotation autour de l'axe de rotation (9) dirigé sur l'objet à examiner (1), décrit en même temps un arc (10), par exemple un arc de cercle, le long du contour de l'objet à examiner (1) (figure 1).

4. Appareil de tomographie par ultrasons selon la revendication 2, caractérisé par le fait que le système de balayage sagittal comporte un détecteur sagittal (30) qui, pendant sa rotation autour de l'axe de rotation (9) dirigé sur l'objet à examiner (1), est susceptible d'être déplacé le long d'une droite (34) qui est parallèle à l'axe de rotation (9) (figure 4).

5. Appareil de tomographie par ultrasons selon la revendication 2, caractérisé par le fait que le système de balayage sagittal comporte un détecteur sagittal (40) qui, pendant sa rotation autour de l'axe de rotation (9) dirigé sur l'objet à examiner (1), est disposé de manière à pouvoir être déplacé le long d'une ligne (44) qui est perpendiculaire à l'axe de rotation (9) et qui se situe à un niveau par rapport à l'objet à examiner (1), qui peut être donné à l'avance (figure 5).

6. Appareil de tomographie par ultrasons selon l'une des revendications 1 à 5, caractérisé par le fait que le système d'émission/de réception par ultrasons comporte plusieurs détecteurs sagittaux (30, 40) qui se déplacent, section par section, suivant des lignes horizontales et verticales (34; 44) (figure 5).

7. Appareil de tomographie selon l'une des revendications 1 à 6, caractérisé par le fait que le système de balayage sagittal (5; 30; 40) comporte plusieurs détecteurs sectoriels du type mécanique.

## Claims

1. Ultrasonic tomography apparatus comprising an ultrasonic transmitting/receiving system designed for scanning an object under examination (1) along lines from different angular directions, and rotatable about a rotary axis (9) pointed towards the object under examination (1), characterised in that the ultrasonic transmitting/receiving system is a Sagittal scanning plane system (5; 30; 40) having transmitting/receiving directions fanning out in each Sagittal scanning plane (15, 16, 17) so as to be sector-shaped and is assigned a timer gating circuit (23a to 23n) for section-wise detection of signal data during predeterminable times ($t_1$ to $t_n$).

2. Ultrasonic tomography apparatus as claimed in Claim 1, characterised in that the Sagittal scanning system (5; 30; 40) comprises at least one Sagittal scanner displaceable at a predetermined distance on a path (10; 34; 44) along the contour of the object under examination (1).

3. Ultrasonic tomography apparatus as claimed in Claim 2, characterised in that the Sagittal scanning system comprises a Sagittal scanner (5) which during its rotation about the rotary axis (9), directed towards the object under examination (1), simultaneously describes an arc (10), e.g. a circular arc along the contour of the object under examination (1) (Figure 1).

4. Ultrasonic tomography apparatus as claimed in Claim 1, characterised in that the Sagittal scanning system comprises a Sagittal scanner (30) which is longitudinally displaceable on a straight line (34) parallel to the rotary axis (9) during its rotation about the rotary axis (9) directed towards the object under examination (1).

5. Ultrasonic tomography apparatus as claimed in Claim 2, characterised in that the Sagittal scanning system comprises a Sagittal scanner (40) which is displaceably arranged along a line (44) at right angles to the rotary axis (9) and a predeterminable vertical distance from the object under examination (1) during its rotation about the rotary axis (9) directed towards the object under examination (1) (Figure 5).

6. Ultrasonic tomography apparatus as claimed in any one of Claims 1 to 5, characterised in that the ultrasonic transmitting/receiving system comprises a plurality of Sagittal scanners (30, 40) which move section by section in horizontal and vertical lines (34; 44) (Figure 5).

7. Ultrasonic tomography apparatus as claimed in any one of Claims 1 to 6, characterised in that the Sagittal scanning system (5; 30; 40) comprises mechanical sector scanners.

FIG 1

FIG 2

FIG 3

FIG 4

FIG 5